# EUROPÄISCHE PATENTSCHRIFT

(11) **EP 2 389 097 B1**
(45) Veröffentlichungstag und Bekanntmachung des Hinweises auf die Patenterteilung: **26.11.2014**
(21) Anmeldenummer: 10700410.3
(22) Anmeldetag: 14.01.2010
(51) Int. Cl.: A61B 5/1455, G01N 33/487, G01N 33/00, A61B 5/151, A61B 5/145

(54) **SYSTEM UND VERFAHREN ZUR ANALYSE EINER KÖRPERFLÜSSIGKEIT**
System and method for analysing a body fluid
Système et procédé destinés à l'analyse d'un liquide corporel

(30) Priorität: 16.01.2009 EP 09150807
(43) Veröffentlichungstag der Anmeldung: 30.11.2011
(73) Patentinhaber: F. Hoffmann-La Roche AG, 4070 Basel (CH); Roche Diagnostics GmbH, 68305 Mannheim (DE)
(72) Erfinder: ZIMMER, Volker, 54497 Morbach (DE); PETRICH, Wolfgang, 76669 Bad Schönborn (DE)
(74) Vertreter: Pfiz, Thomas
(86) Internationale Anmeldenummer: PCT/EP2010/050395
(87) Internationale Veröffentlichungsnummer: WO 2010/081848

(56) Entgegenhaltungen:
- WO-A-2005/006985
- WO-A-2005/084545
- WO-A-2005/084546
- US-A- 5 801 057
- US-A1- 2005 137 471

## Beschreibung

Die Erfindung betrifft ein System bzw. eine Vorrichtung zur Analyse einer Körperflüssigkeit gemäß dem Oberbegriff des Patentanspruchs 1. Die Erfindung betrifft weiter ein entsprechendes Analyseverfahren.

Ein ähnliches System für Blutzuckertests ist aus der WO 2005/084530 bekannt, in der eine integrierte Kombination eines Sammelelements mit einer analytischen Testzone beschrieben ist, wobei nach dem Sammelvorgang ein Probentransfer durch Verformung eines die Flüssigkeit führenden Kanals erfolgen soll. Dort findet sich allerdings kein Hinweis auf die Randbedingungen des Probentransfers insbesondere für vor und/oder nach der Messung körperlich voneinander getrennter Bauteile. Weiterhin können sich durch die integrierte Anordnung Probleme hinsichtlich der sterilen Bereithaltung und Entsorgung der Verbrauchsteile ergeben.

Die WO 2005/084546 offenbart ein Blutzuckeranalysesystem mit einem Sammelelement, das eine rasterförmige Anordnung von Sammellöchern aufweist, aus denen Körperflüssigkeit auf eine Testzone vertikal übertragen wird, wobei der benetzte Bereich etwa dem Durchmesser der Löcher entspricht, so dass sich das Lochmuster zur Detektionsseite hin abbildet.

Ausgehend hiervon liegt der Erfindung die Aufgabe zugrunde, die im Stand der Technik bekannten Erzeugnisse und Verfahren weiter zu entwickeln und eine auch hinsichtlich der Bereitstellung/Entsorgung der Verbrauchsteile sowie reproduzierbarer Messergebnisse optimierte Konfiguration anzugeben.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von der Erkenntnis aus, dass der Messerfolg entscheidend davon abhängt, dass das Testelement über die Messdauer im Wesentlichen stets mit der Probenflüssigkeit beaufschlagt bleibt. Dementsprechend wird vorgeschlagen, dass die Transfereinrichtung einen Spreitbereich des Testelements während des Messintervalls dauerhaft in flächigen Kontakt mit der im Reservoir befindlichen Körperflüssigkeit bringt, so dass eine Flüssigkeitssäule auf einer Nachweisfläche des Testelements erhalten bleibt, wobei das Flüssigkeitsvolumen des Reservoirs und die Größe des Spreitbereichs so bestimmt ist, dass die Flüssigkeitssäule über dem Bereich der optisch abgetasteten Nachweisfläche für die Dauer des Messintervalls eine Mindesthöhe nicht unterschreitet, wobei die Mindesthöhe der Flüssigkeitssäule mehr als 50µm beträgt und das Reservoir zur Aufnahme von Körperflüssigkeit in Form einer über ihre Länge halboffenen Kapillare ausgebildet ist. Durch ein hinreichendes Flüssigkeitsvolumen auf der Nachweisfläche laufen Reaktionsprozesse und insbesondere Diffusionsvorgänge gleichförmig ab, ohne dass Schichtdickeneffekte auch im Hinblick auf Toleranzen noch eine wesentliche Rolle spielen. Die Flüssigkeitssäule steht auf bzw. über der Oberfläche des Testelements und weist somit einen Flüssigkeitsspiegel in einem durch das Sammelelement begrenzten Raum auf. Der Einsatz gesonderter Elemente für die Flüssigkeitsgewinnung und den analytischen Nachweis bedingt ein aktives Zusammenbringen, so dass sich auch der Reaktionsstart genau definieren lässt. Zugleich wird im Vorfeld ein vereinfachtes Handling auch hinsichtlich Sterilisierung und Bevorratung gewährleistet, während durch die Trennung nach der Messung die Entsorgung in einer Form erfolgen kann, die der vorherigen Bereitstellung entspricht, so dass auch die Magazinierung erleichtert wird. Nicht zuletzt wird in dieser Konfiguration eine Kontamination des Sammelelements mit der Testchemie und somit eine Gefährdung beim Körperkontakt oder eine Verminderung von hydrophilen Eigenschaften vermieden.

Erfindungsgemäß ist das Flüssigkeitsvolumen des Reservoirs so bestimmt, dass die Flüssigkeitssäule über der Nachweisfläche für die Dauer des Messintervalls eine Mindesthöhe nicht unterschreitet. Hierbei ist es günstig, wenn die Mindesthöhe der Flüssigkeitssäule mehr als 50µm beträgt. Dadurch lassen sich auch produktionstechnische Toleranzen ausgleichen, und die Mindesthöhe, unterhalb derer Diffusionsprozesse noch eine entscheidende Rolle spielen, ist zuverlässig überschritten.

Grundsätzlich ist das Testelement größer als die tatsächlich erfasste Nachweisfläche. Um eine Ortsabhängigkeit zu vermeiden, sollte das Testfeld dazu ausgebildet sein, die Körperflüssigkeit während des Messintervalls in einem Spreitbereich flächig zu verteilen, wobei zu gewährleisten ist, dass die Größe des Spreitbereichs und das Volumen des Reservoirs aufeinander abgestimmt sind, so dass während des Messintervalls über der Nachweisfläche eine Flüssigkeitssäule erhalten bleibt.

Auch in diesem Zusammenhang ist es von Vorteil, wenn das Reservoir eine Tiefe zwischen 50 und 150 µm, eine Breite zwischen 50 und 150 µm und eine Länge von mehr als 1 mm, vorzugsweise etwa 2 mm aufweist. Für eine zuverlässige Erfassung bei gleichzeitiger Begrenzung der erforderlichen Probenmenge ist es auch günstig, wenn die Nachweisfläche eine Größe im Bereich von 0,1 mm² bis 1 mm² aufweist.

Messtechnisch ist es vorteilhaft, wenn das analytspezifische Messsignal in einem Zeitbereich von 0 bis 15 s nach der Herstellung der fluidischen Verbindung zwischen dem Sammel- und Testelement erfasst wird. Hierbei kann as Ende des Messintervalls dadurch bestimmt sein, dass die Änderung des Messsignals in der Zeiteinheit einen vorgegebenen Wert erreicht

Eine besonders bevorzugte Ausführung sieht vor, dass das Sammelelement und Testelement in einem Ausgangszustand als gesonderte Bauteile voneinander beabstandet sind, also körperlich voneinander getrennt sind, und dass die fluidische Verbindung durch Abstandsreduzierung unter aktiver Bewegung des Sammelelements und/oder des Testelements herstellbar ist. Durch die anfängliche Trennung der Bauteile ergeben sich auch vielfache Vorteile hinsichtlich vereinfachter Fertigungs- und Einsatzmöglichkeiten.

Um eine Probenentnahme unabhängig von der Testchemie zu ermöglichen, ist es von Vorteil, wenn die Flüssigkeitsaufnahme durch einen Stechvorgang erfolgt, und wenn die fluidische Verbindung des Sammelelements mit dem Testelement erst nach dem Stechvorgang hergestellt wird.

Vorteilhafterweise ist das Reservoir durch eine Kapillarstruktur, insbesondere einen linearen Kapillarkanal oder Kapillarspalt gebildet. Weiterhin ist es vorteilhaft, wenn die Nachweisfläche als Trockenchemieschicht insbesondere auf Enzymbasis für eine photometrische Erfassung ausgebildet ist, und wenn das Sammelelement zumindest im Bereich des Reservoirs mit einer hydrophilen Beschichtung versehen ist.

Gebrauchsvorteile ergeben sich auch dadurch, dass das Sammelelement und das Testelement nach dem Messintervall beispielsweise für eine separate Entsorgung als Einwegteile wieder voneinander trennbar sind. Dies erfolgt bevorzugt mittels der Transfereinrichtung, weiche die anfänglich getrennten Bauteile in einen definierten Kontaktzustand für die Messung bringt, der allerdings nach Abschluss der Messung nicht mehr benötigt wird.

In verfahrensmäßiger Hinsicht wird die eingangs genannte Aufgabe durch die im Patentanspruch 11 genannten Schritte gelöst.

Im Folgenden wird die Erfindung anhand eines in der Zeichnung schematisch dargestellten Ausführungsbeispiels näher erläutert. Es zeigen:
- Fig. 1: ein diagnostisches Analysesystem in einer vereinfachten schaubildlichen Darstellung;
- Fig. 2: verschiedene Relativstellungen eines Sammelelements und eines Testelements des Analysesystems während des Messablaufs im Querschnitt;
- Fig. 3: ein Messsignal als Funktion der Zeit; und
- Fig. 4: ein Messdiagramm eines Vergleichsversuchs.

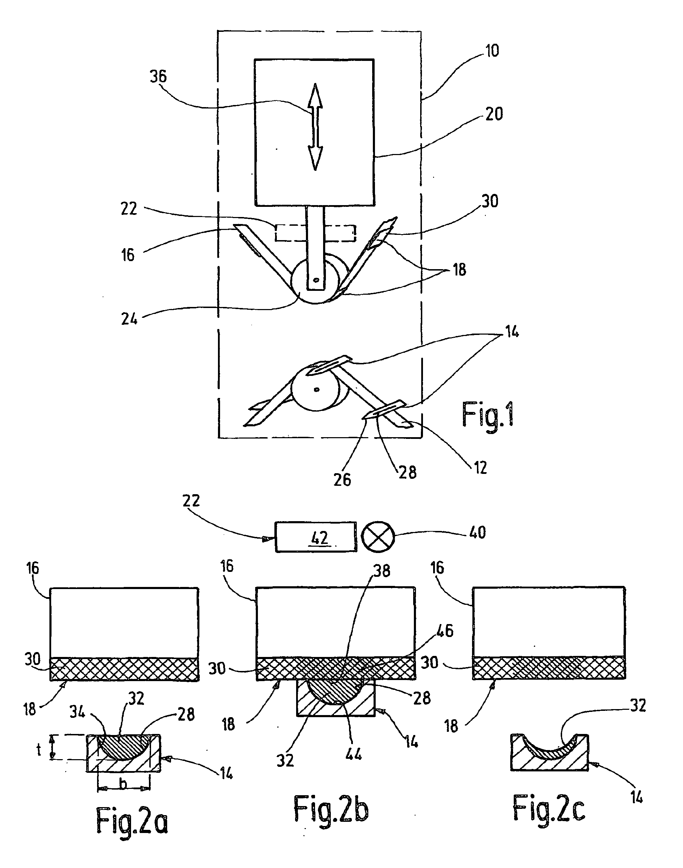

Das in Fig. 1 veranschaulichte Analysesystem 10 umfasst in Form eines Handgeräts zur Durchführung von Blutzuckertests ein Trägerband 12 mit einer Vielzahl von Sammelelementen 14 für Körperflüssigkeit (Blut bzw. Gewebeflüssigkeit) sowie ein gesondertes Trägerband 16 zur Magazinierung einer entsprechenden Anzahl von Testelementen 18, eine Probentransfereinrichtung 20 und eine Detektionseinheit 22. Durch Bandtransport kann jeweils ein Sammelelement 14 und ein Testelement 18 an einer Bandumlenkung 24 für einen Einmalgebrauch bereitgestellt werden. Die Probentransfereinrichtung 20 ist dabei so eingerichtet, dass während eines Messintervalls das aktive Testelement 18 stets mit Körperflüssigkeit beaufschlagt bleibt.

Die Trägerbänder 12, 16 mit den darauf befindlichen Sammelelementen 14 bzw. Testelementen 18 können in Form von jeweiligen Bandkassetten bereitgestellt werden, so dass eine gesonderte Bevorratung und Entsorgung möglich ist. Auch andere Formen einer gesonderten Magazinierung beispielsweise in Stapelform sind denkbar. Durch die gesonderte Anordnung ist es möglich, die Sammelelemente 14 unabhängig von den Testelementen 18 beispielsweise durch energiereiche Bestrahlung zu sterilisieren. Zudem kann eine gesonderte stoffdichte Verpackung auch längere Lagerzeiten ohne Qualitätsnachteile garantieren.

Die Sammelelemente 14 können dazu ausgebildet sein, die Körperflüssigkeit direkt vor Ort aufzunehmen. Zu diesem Zweck können die Sammelelemente 14 jeweils eine Spitze 26 für einen Hauteinstich und ein Reservoir zur Aufnahme von Körperflüssigkeit in Form einer über ihre Länge halboffenen Kapillare 28 aufweisen. Grundsätzlich ist es auch möglich, Sammelelemente als Zwischenträger mittelbar mit der Körperflüssigkeit zu beaufschlagen.

Die Testelemente 18 sind als Testfelder mit einer Trockenchemieschicht 30 auf Enzymbasis für einen optischen Glucosenachweis versehen. Der Nachweis anhand einer Farbänderung kann durch transparente Strukturen hindurch mittels einer photometrischen Detektionseinheit 22 erfolgen, wie es beispielsweise in der EP-A 1760469 näher beschrieben ist.

Fig. 2 veranschaulicht drei verschiedene Verfahrensstadien beim Einsatz der einander paarweise zugeordneten Sammel- und Testelemente.

In einem Ausgangszustand gemäß Fig. 2a ist das Sammelelement 14 im Abstand von dem Testelement 18 körperlich getrennt. Die Kapillare 28 ist mit Körperflüssigkeit 32 gefüllt. Um die Flüssigkeitsaufnahme zu erleichtern, kann die Oberfläche des Sammelelements 14 zumindest im Bereich der Kapillare 28 mit einer hydrophilen Beschichtung 34 versehen sein. Typische Abmessungen der Kapillare 28 für den besonderen Einsatzzweck betragen ca. 120 µm in der Breite b, ca. 80 µm in der Tiefe t und ca. 2 mm in der Länge.

In einem nächsten Schritt gemäß Fig. 2b erfolgt die Messwerterfassung. Hierzu wird mittels eines durch Doppelpfeil 36 in Fig. 1 symbolisierten Aktors der Transfereinrichtung 20 unter Abstandsreduzierung eine fluidische Verbindung zwischen dem Sammelelement 14 und dem Testelement 18 für einen Probentransfer hergestellt. Dies erfolgt derart, dass die Kapillare 28 öffnungsseitig auf die Trockenchemieschicht 30 aufgesetzt wird, wobei der mit der Körperflüssigkeit 32 beaufschlagte Bereich eine Nachweisfläche 38 mit einem Flächeninhalt von etwa 0,2 mm² für die photometrische Messwerterfassung bildet. Die Messung erfolgt im Kontaktzustand des Sammelelements 14 rückseitig durch das transparente Trägerband 16 hindurch. Zu diesem Zweck weist die Detektionseinheit 22 eine Lichtquelle 40 und einen Lichtempfänger 42 in einer reflektometrischen Anordnung auf.

Durch die Aufrechterhaltung des Kontaktzustands ist sichergestellt, dass während des Messintervalls eine durch die Kapillare 28 begrenzte Flüssigkeitssäule 44 auf der Nachweisfläche 38 erhalten bleibt. Die Flüssigkeitssäule 44 befindet sich somit in einem durch das Sammelelement 14 begrenzten Raum über der benetzten Oberfläche des Testelements 18. Die Dimensionen der Kapillare 28 sind dabei so gewählt, dass eine Mindesthöhe der Flüssigkeitssäule 44 von ca. 50 µm zumindest über dem Bereich der optisch abgetasteten Nachweisfläche 38 nicht unterschritten wird. Hierbei ist durch ein hinreichendes Kapillarvolumen als Reservoir auch berücksichtigt, dass die Trockenchemieschicht 30 mit einer spreitenden Oberfläche versehen ist, welche die Körperflüssigkeit 32 in einem Spreitbereich 46 flächig verteilt.

Wie aus Fig. 2c ersichtlich, ist die Transfereinrichtung 20 auch dazu ausgebildet, das Sammelelement 14 und das Testelement 18 nach der Messung wieder aktiv mittels Aktor 36 voneinander zu trennen. Auf diese Weise wird eine getrennte Entsorgung entsprechend der Ausgangssituation ermöglicht. Bei dem gezeigten Ausführungsbeispiel erfolgt dies in Form von getrennten Bändern 12, 16, die eine Re-Magazinierung erheblich vereinfachen. Der Benutzer kann somit Verbrauchseinheiten in das Gerät 10 einsetzen, die ohne aufwändige Handhabung eine Vielzahl von Tests ermöglichen.

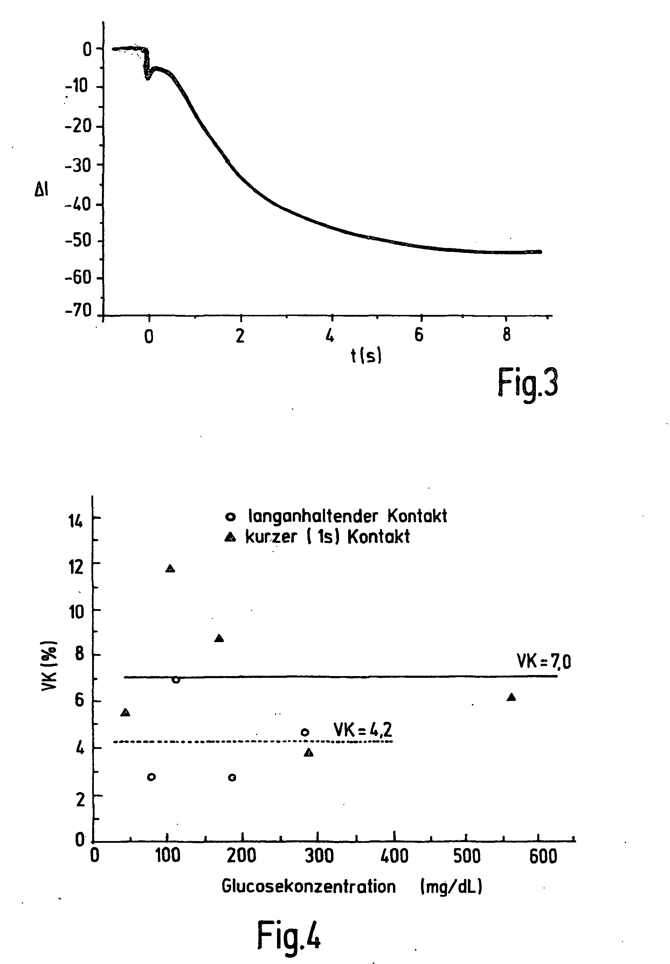

Fig. 3 zeigt den zeitlichen Verlauf eines mittels der Detektionseinheit 22 erfassten Signals. Das Messintervall umfasst zumindest ein solches Zeitfenster, in welchem die Beobachtung eines auf einer Reaktion des Analyten (Glucose) beruhenden Signalverlaufs möglich ist. Dies kann mit dem Ziel erfolgen, die Reaktionskinetik zu analysieren, oder aber direkt einen Konzentrationswert zu ermitteln.

Der Nachweis beruht auf einer durch die analytspezifische Reaktion bedingten Farbänderung der Nachweisfläche 38, die als Grauwertverlauf Δl in Remission erfasst wird. Während zunächst noch im getrennten Zustand ein Leerwert bzw. Nullwert beobachtet wird, tritt mit dem Kontakt gemäß Fig. 2b ein analytspezifischer Signalabfall ein, der sich schließlich einem für die Glukosekonzentration charakteristischen Endwert nähert. Die Dauer des Messintervalls, in welchem das analytspezifische Signal erfasst wird, kann durch einen vorgegebenen Grenzwert der Signaländerung pro Zeiteinheit definiert werden. Beispielsweise könnte die Messung abgebrochen werden, wenn sich das Signal um weniger als 3% in der Sekunde ändert. Die Messdauer liegt dann üblicherweise unter 8s.

In einem Vergleichsversuch wurden Messungen mit erfindungsgemäßem lang anhaltendem Kontakt zwischen Sammelelement und Testelement und Messungen mit Kurzzeitkontakt (ca. 1 s) nach Befüllung der Kapillare mit Testflüssigkeit verschiedener Glucosekonzentration durchgeführt. Fig. 4 zeigt die Streuung der Versuchsergebnisse in Form des Varianzkoeffizienten über der Glucosekonzentration. Daraus ergibt sich ein signifikant niedrigerer mittlere Varianz von 4,2% für den langanhaltenden Kontakt im Vergleich zu einer mittleren Varianz von 7% für den Kurzzeitkontakt. Durch die Aufrechterhaltung der Flüssigkeitssäule 32 laufen die Reaktionsprozesse also reproduzierbarer ab. Aufgrund einer vergleichsweise großen Mindesthöhe der Flüssigkeitsschicht lässt sich sicherstellen, dass Diffusionsvorgänge weitgehend unabhängig von der Schichthöhe verlaufen, während bei einem Kurzzeitkontakt gleichsam in Form eines Abklatschens der Flüssigkeit auf der Trockenchemieschicht 30 die Prozesse lokal unterschiedlich verlaufen können.

## Patentansprüche

1. System zur Analyse einer Körperflüssigkeit mit
einem die Körperflüssigkeit (32) in einem Reservoir (28) aufnehmenden Sammelelement (14),
einem zum Nachweis eines Analyten in der Körperflüssigkeit (32) ausgebildeten Testelement (18),
einer Transfereinrichtung (20) zur Herstellung einer fluidischen Verbindung zwischen dem Sammelelement (14) und dem Testelement (18), und einer während eines Messintervalls ein analytspezifisches Messsignal an dem Testelement (18) erfassenden Detektionseinheit (22),
wobei die Transfereinrichtung (20) dazu eingerichtet ist, das Testelement (18) und das Sammelelement (14) nach dem Messintervall voneinander körperlich zu trennen,
**dadurch gekennzeichnet, dass**
die Transfereinrichtung (20) einen Spreitbereich (46) des Testelements (18) während des Messintervalls dauerhaft in flächigen Kontakt mit der im Reservoir (28) befindlichen Körperflüssigkeit (32) bringt, so dass eine Flüssigkeitssäule (44) auf einer Nachweisfläche (38) des Testelements (18) erhalten bleibt,
wobei das Flüssigkeitsvolumen des Reservoirs (28) und die Größe des Spreitbereichs (46) so bestimmt ist, dass die Flüssigkeitssäule (44) über dem Bereich der optisch abgetasteten Nachweisfläche (38) für die Dauer des Messintervalls eine Mindesthöhe nicht unterschreitet, wobei die Mindesthöhe der Flüssigkeitssäule (44) mehr als 50µm beträgt und das Reservoir (28) zur Aufnahme von Körperflüssigkeit in Form einer über ihre Länge halboffenen Kapillare (28) ausgebildet ist.

2. System nach Anspruch 1, **dadurch gekennzeichnet, dass** das Reservoir (28) eine Tiefe zwischen 50 und 150 µm, eine Breite zwischen 50 und 150 µm und eine Länge von mehr als 1 mm, vorzugsweise etwa 2 mm aufweist.

3. System nach Anspruch 1 oder 2, **dadurch gekennzeichnet, dass** die Nachweisfläche (38) eine Größe im Bereich von 0,1 mm² bis 1 mm² aufweist.

4. System nach einem der Ansprüche 1 bis 3, **dadurch gekennzeichnet, dass** das analytspezifische Messsignal in einem Zeitbereich von 0 bis 15 s nach der Herstellung der fluidischen Verbindung zwischen dem Sammel- und Testelement (14,18) erfasst wird.

5. System nach einem der Ansprüche 1 bis 4, **dadurch gekennzeichnet, dass** das Ende des Messintervalls dadurch bestimmt ist, dass die Änderung des Messsignals in der Zeiteinheit einen vorgegebenen Wert erreicht.

6. System nach einem der Ansprüche 1 bis 5, **dadurch gekennzeichnet, dass** das Sammelelement (14) und Testelement (18) in einem Ausgangszustand als gesonderte Bauteile voneinander beabstandet sind, und dass die fluidische Verbindung unter Bewegung des Sammelelements (14) und/oder des Testelements (18) herstellbar ist.

7. System nach einem der Ansprüche 1 bis 6, **dadurch gekennzeichnet, dass** die Flüssigkeitsaufnahme durch einen Stechvorgang erfolgt, und dass die fluidische Verbindung des Sammelelements (14) mit dem Testelement (18) erst nach dem Stechvorgang hergestellt wird.

8. System nach einem der Ansprüche 1 bis 7, **dadurch gekennzeichnet, dass** das Reservoir (28) durch eine Kapillarstruktur, insbesondere einen vorzugsweise einseitig offenen linearen Kapillarkanal oder Kapillarspalt gebildet ist.

9. System nach einem der Ansprüche 1 bis 8, **dadurch gekennzeichnet, dass** die Nachweisfläche (38) durch eine Trockenchemieschicht (30) insbesondere auf Enzymbasis für eine photometrische Erfassung gebildet ist.

10. System nach einem der Ansprüche 1 bis 9, **dadurch gekennzeichnet, dass** das Sammelelement (14) zumindest im Bereich des Reservoirs (28) mit einer hydrophilen Beschichtung (34) versehen ist.

11. Verfahren zur Analyse einer Körperflüssigkeit bei welchem
eine fluidische Verbindung zwischen einem die Körperflüssigkeit (32) in einem Reservoir (28) enthaltenden Sammelelement (14) und einem zum Nachweis eines Analyten in der Körperflüssigkeit (32) ausgebildeten Testelement (18) hergestellt wird,
während eines Messintervalls ein analytspezifisches Messsignal an dem Testelement (18) erfasst wird,
das Testelement (18) und das Sammelelement (14) nach dem Messintervall voneinander getrennt werden,
**dadurch gekennzeichnet, dass**
- ein Spreitbereich (46) des Testelements (18) während des Messintervalls dauerhaft in flächigen Kontakt mit der im Reservoir (28) befindlichen Körperflüssigkeit (32) gebracht wird, so dass eine Flüssigkeitssäule (44) auf einer Nachweisfläche (38) des Testelements (18) erhalten bleibt,
- wobei das Flüssigkeitsvolumen des Reservoirs (28) so bestimmt wird, dass die Flüssigkeitssäule (44) über dem Bereich der optisch abgetasteten Nachweisfläche (38) für die Dauer des Messintervalls eine Mindesthöhe nicht unterschreitet, wobei die Mindesthöhe der Flüssigkeitssäule (44) mehr als 50µm beträgt, und
- das Reservoir (28) zur Aufnahme von Körperflüssigkeit in Form einer über ihre Länge halboffenen Kapillare (28) ausgebildet ist.

## Claims

1. System for analyzing a body fluid comprising
a collecting element (14) which receives the body fluid (32) in a reservoir (28),
a test element (18) designed to detect an analyte in the body fluid (32),
a transfer device (20) for making a fluidic connection between the collecting element (14) and the test element (18), and
a detection unit (22) which detects an analyte-specific measurement signal on the test element (18) during a measurement interval, wherein the transfer device (20) is configured to physically separate the test element (18) and the collecting element (14) from one another after the measurement interval,
**characterized in that**
the transfer device (20) permanently brings a spreading area (46) of the test element (18) into two-dimensionally contact with the body fluid (32) located in the reservoir (28) during the measurement interval so that a liquid column (44) is maintained on a detection area (38) of the test element (18),
wherein the liquid volume of the reservoir (28) and the size of the spreading area (46) is defined such that the liquid column (44) over the region of the optically scanned detection area (38) does not fall below a minimum height for the duration of the measurement interval, wherein the minimum height of the liquid column (44) is more than 50 µm and the reservoir (28) for receiving body fluid is designed in the form of a longitudinally semi-open capillary (28).

2. System according to claim 1, **characterized in that** the reservoir (28) has a depth between 50 and 150 µm, a width between 50 and 150 µm and a length of more than 1 mm, preferably about 2 mm.

3. System according to claim 1 or 2, **characterized in that** the detection area (38) has a size in the range of 0.1 mm² to 1 mm².

4. System according to one of the claims 1 to 3, **characterized in that** the analyte-specific measurement signal is detected within a period of 0 to 15 s after making the fluidic connection between the collecting element and test element (14, 18).

5. System according to one of the claims 1 to 4, **characterized in that** the end of the measurement interval is determined as being when the change in the measurement signal per unit of time has reached a predefined value.

6. System according to one of the claims 1 to 5, **characterized in that** the collecting element (14) and test element (18) are spaced apart as separate components in an initial state and that the fluidic connection can be made while actively moving the collecting element (14) and/or the test element (18).

7. System according to one of the claims 1 to 6, **characterized in that** the fluid is taken up by a lancing process and that the fluidic connection of the collecting element (14) to the test element (18) is not made until after the lancing process.

8. System according to one of the claims 1 to 7, **characterized in that** the reservoir (28) is formed by a capillary structure, in particular a linear capillary channel or capillary gap which is preferably open on one side.

9. System according to one of the claims 1 to 8, **characterized in that** the detection area (38) is formed by a dry chemistry layer (30) in particular based on enzymes for a photometric detection.

10. System according to one of the claims 1 to 9, **characterized in that** the collecting element (14) is provided with a hydrophilic coating (34) at least in the area of the reservoir (28).

11. Method for analyzing a body fluid in which
a fluidic connection is made between a collecting element (14) containing the body fluid (32) in a reservoir (28) and a test element (18) designed to detect an analyte in the body fluid (32),
an analyte-specific measurement signal is detected on the test element (18) during a measurement interval,
the test element (18) and the collecting element (14) are separated from one another after the measurement interval,
**characterized in that**
- a spreading area (46) of the test element (18) is permanently brought into two-dimensionally contact with the body fluid (32) located in the reservoir (28) during the measurement interval so that a column of liquid (44) is maintained on a detection area (38) of the test element (18),
- wherein the liquid volume of the reservoir (28) is defined such that the liquid column (44) over the region of the optically scanned detection area (38) does not fall below a minimum height for the duration of the measurement interval, wherein the minimum height of the liquid column (44) is more than 50 µm, and
- the reservoir (28) for receiving body fluid is designed in the form of a longitudinally semi-open capillary (28).

## Revendications

1. Système pour l'analyse d'un liquide corporel, comprenant :
un élément de récolte (14) qui réceptionne le liquide corporel (32) dans un réservoir (28) ;
un élément de test (18) réalisé pour le décèlement d'un analyte dans le liquide corporel (32) ;
un mécanisme de transfert (20) pour l'obtention d'une liaison par fluide entre l'élément de récolte (14) et l'élément de test (18) ; et
une unité de détection (22) qui enregistre, au cours d'un intervalle de mesure, un signal de mesure spécifique à un analyte, sur l'élément de test (18) ;
le mécanisme de transfert (20) étant conçu de façon à séparer matériellement l'un de l'autre l'élément de test (18) et l'élément de récolte (14) après l'intervalle de mesure ;
**caractérisé en ce que**
le mécanisme de transfert (20) amène une zone d'étalement (46) de l'élément de test (18) au cours de l'intervalle de mesure en contact superficiel permanent avec le liquide corporel (32) qui se trouve dans le réservoir (28), de telle sorte que l'on maintient une colonne de liquide (44) sur une surface de décèlement (38) de l'élément de test (18) ;
le volume de liquide du réservoir (28) et la dimension de la zone d'étalement (46) étant déterminés de telle sorte que la colonne de liquide (44) ne dépasse pas vers le bas une hauteur minimale sur la zone de la surface de décèlement (38) enregistrée par voie optique, pendant la durée de l'intervalle de mesure, la hauteur minimale de la colonne de liquide (44) s'élevant à une valeur supérieure à 50 µm et le réservoir (28) pour la réception du liquide corporel étant réalisé sous la forme d'un capillaire (28) demi ouvert sur sa longueur.

2. Système selon la revendication 1, **caractérisé en ce que** le réservoir (28) présente une profondeur entre 50 et 150 µm, une largeur entre 50 et 150 µm et une longueur supérieure à 1 mm, de préférence d'environ 2 mm.

3. Système selon la revendication 1 ou 2, **caractérisé en ce que** la surface de décèlement (38) présente une dimension dans la plage de 0,1 mm² à 1 mm².

4. Système selon l'une quelconque des revendications 1 à 3, **caractérisé en ce que** le signal de mesure spécifique à un analyte est enregistré dans un intervalle de temps de 0 à 15 secondes, après l'obtention de la liaison par fluide entre l'élément de récolte et l'élément de test (14, 18).

5. Système selon l'une quelconque des revendications 1 à 4, **caractérisé en ce qu'**on détermine la fin de l'intervalle de mesure par le fait que la modification du signal de mesure atteint une valeur prédéfinie dans l'unité de temps.

6. Système selon l'une quelconque des revendications 1 à 5, **caractérisé en ce que** l'élément de récolte (14) et l'élément de test (18) sont disposés à l'écart l'un de l'autre sous la forme d'éléments séparés à l'état de départ et **en ce qu'**on peut mettre en oeuvre la liaison par fluide en déplaçant l'élément de récolte (14) et/ou l'élément de test (18).

7. Système selon l'une quelconque des revendications 1 à 6, **caractérisé en ce que** la réception du liquide a lieu via un processus de piqûre et **en ce qu'**on met en oeuvre la liaison par fluide de l'élément de récolte (14) avec l'élément de test (18) seulement après le processus de piqûre.

8. Système selon l'une quelconque des revendications 1 à 7, **caractérisé en ce que** le réservoir (28) est formé par une structure capillaire, en particulier une fente capillaire ou un canal capillaire linéaire, de préférence ouvert d'un côté.

9. Système selon l'une quelconque des revendications 1 à 8, **caractérisé en ce que** la surface de décèlement (38) est formée par une couche de chimie sèche (30) en particulier à base enzymatique pour un enregistrement photométrique.

10. Système selon l'une quelconque des revendications 1 à 9, **caractérisé en ce que** l'élément de récolte (14) est muni d'une enduction hydrophile (34) au moins dans la zone du réservoir (28).

11. Procédé pour l'analyse d'un liquide corporel dans lequel :
on met en oeuvre une liaison par fluide entre un élément de récolte (14) contenant le liquide corporel (32) dans un réservoir (28) et un élément de test (18) réalisé pour le décèlement d'un analyte dans le liquide corporel (32) ;
au cours d'un intervalle de mesure, on enregistre un signal de mesure spécifique à un analyte, sur l'élément de test (18) ;
on sépare l'un de l'autre l'élément de test (18) et l'élément de récolte (14) après l'intervalle de mesure ;
**caractérisé en ce que**
- on amène une zone d'étalement (46) de l'élément de test (18) au cours de l'intervalle de mesure en contact superficiel permanent avec le liquide corporel (32) qui se trouve dans le réservoir (28), de telle sorte que l'on maintient une colonne de liquide (44) sur une surface de décèlement (38) de l'élément de test (18) ;
- le volume de liquide du réservoir (28) est déterminé de telle sorte que la colonne de liquide (44) ne dépasse pas vers le bas une hauteur minimale sur la zone de la surface de décèlement (38) enregistrée par voie optique, pendant la durée de l'intervalle de mesure, la hauteur minimale de la colonne de liquide (44) s'élevant à une valeur supérieure à 50 µm ; et
- le réservoir (28) pour la réception du liquide corporel étant réalisé sous la forme d'un capillaire (28) demi ouvert sur sa longueur.
